# EUROPEAN PATENT SPECIFICATION

(11) **EP 0 609 214 B1**
(45) Date of publication and mention of the grant of the patent: **26.02.1997**
(21) Application number: 92905217.3
(22) Date of filing: 18.12.1991
(51) Int. Cl.: A61B 19/04, A61F 13/02

(54) **PUNCTURE RESISTANT ARTICLE**
PUNKTIONBESTÄNDIGER GEGENSTAND
ARTICLE RESISTANT A LA PERFORATION

(30) Priority: 02.01.1991 US 636964
(43) Date of publication of application: 10.08.1994
(73) Proprietor: AlliedSignal Inc., Morristown, New Jersey 07962-2245 (US)
(72) Inventor: PHILLIPS, Christopher, Robert 20213 Cox Road, Dinwiddie, VA 23885 (US)
(74) Representative: Brock, Peter William
(86) International application number: US9109580
(87) International publication number: WO9211821

(56) References cited:
- EP-A- 0 197 278
- WO-A-91/17099
- GB-A- 2 207 436
- US-A- 4 375 530
- US-A- 4 413 110
- US-A- 4 858 245
- US-A- 4 864 661
- US-A- 4 966 138

## Description

The present invention relates to a protective tape/patch material which is substantially puncture resistant so as to enable the patch to be placed anywhere on the body, particularly in or around the hands to protect that piece of that body from harm by a sharp object such as a needle, etc.

Protective gloves, fingers, etc., for surgeons are known. For example, US-A-4,858,254 discloses an armor glove finger knit from high strength fibers such as polyamide fiber, polyethylene teraphthalate fiber, and ultrahigh molecular weight polyethylene fibers. US-A-4 864 661 discloses a puncture resistant surgical glove where the puncture resistant patches are adhered to a glove at strategic points to protect a surgeon against injuries due to puncture. US-A-4 966 138 discloses a puncture protector for the body having a flexible backing with adhesive on one side of the backing and a flexible shield member attached to the backing.

However, this sort of glove presents a potential problem in that the protective portions of the glove may not conform to areas in which the surgeon deems himself subject to punctures; or the areas susceptible to puncture may be outside of the area normally covered by the surgeon's glove.

WO91/17099 discloses a system for the disposal of medical waste comprising separable containers for sharps and non-sharps. The sharps container has a restricted opening, to avoid spillage, and is preferably formed of blow-moulded polycarbonate.

This invention is an improvement on a protective puncture resistant flexible patch, whereby the patch can be applied to a vulnerable portion of the hand or body to prevent it from suffering cuts or punctures normally incurred during surgery or other activities wherein the person wearing the patch is susceptible to cuts or punctures in selected portions of the body, characterized in that the thin, semi-flexible puncture-resistant piece is fabricated from extended chain polyethylene (ECPE) fibers which have been subjected to a temperature and at a pressure and for a time sufficient to cause adjacent fibers to partially coalesce and adhere and to deform to form a translucent article substantially free of voids. The protective patch preferably has a puncture resistance of at lest 13.4 kg/cm (75 lbs./in.) as measured by the puncture test specified herein, and preferably a Modulus of Elasticity (MOE) of less than 0.84 x 10⁵ kg/cm² (12 x 10⁵ PSI) preferably less than 0.42 x 10⁵ kg/cm² (6 x 10⁵ PSI) as measured by ASTM Test D-790-86. The ECPE fibers have a tenacity of at least 6.2 dN/tex (7 grams per denier) and a tensile modulus of at least 441.5 dN/tex (500 grams per denier).

Fig. 1 is a view of a protective patch of the type exemplified by this invention.

Fig. 2 is a view taken along lines 2-2 of Fig. 1 showing an edge view of the patch of this invention.

Fig. 3 is a round protective patch of this invention.

Fig. 4 is a schematic showing of the "Puncture Test" specified in this specification.

Fig. 5 is a chart showing comparable flexibilities of the material of this invention and some prior art.

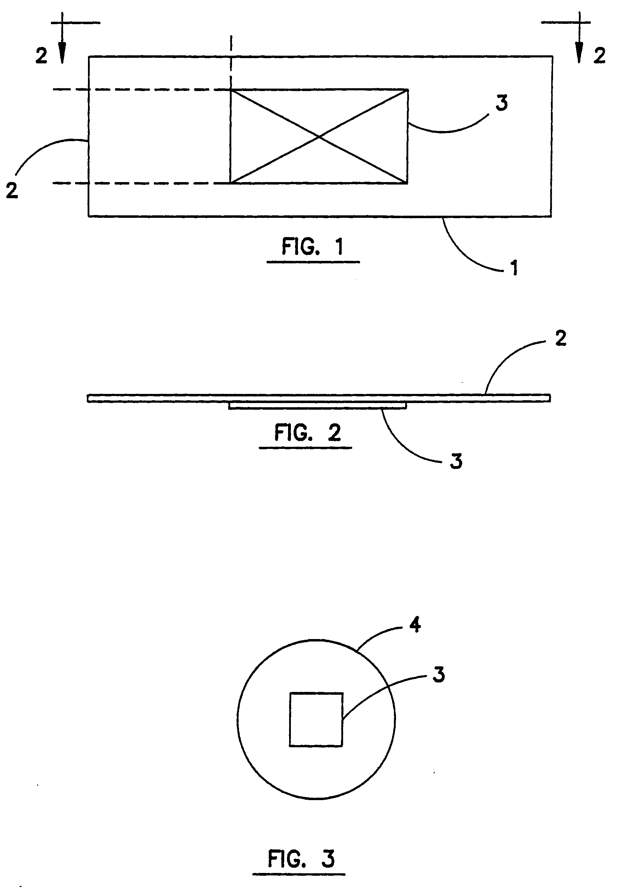

Referring to Fig. 1, the protective patch 1 of this invention is shown as comprising a conventional surgical tape 2 to which is adhered somewhere along the length thereof, preferably in the center, one or more puncture resistant pieces 3. While the protective patch 1 is shown as an elongated article in Fig. 1, it can also be made as a round patch 4 containing a puncture resistant piece 3 as shown in Fig. 3.

The piece 3 is adhered to the tape by conventional adhesives, such as the acrylic adhesive or its equivalents normally used an surgical tape.

Optionally, the puncture resistant flexible piece 3 may be used by itself as the puncture resistant patch merely by applying an adhesive to one side thereof and using it without the tape 2.

One preferred aspect of this invention is the structure of the puncture resistant piece 3 which may be formed from ECPE fibers. This process is described in more detail in US-A-5,135 804 (issued August 4, 1992) entitled Consolidation of Polyethylene Fibrous Networks. This patent discloses that articles can be prepared by applying pressure to a network of ECPE fibers at elevated temperature without substantially impairing the physical properties of the fibers. The process comprises applying pressure to a network of fibers consisting essentially of ECPE fibers having weight average molecular weight at least 500,000, said fibers having a tenacity at least 6.2 dN/tex (7 g/denier), preferably 13.25 to 22.1 dN/tex (15 to 25 g/denier) and tensile modulus at least 441.5 dN/tex (500 g/denier), at a temperature between 100°C and 160°C, sufficient to cause adjacent fibers to adhere. Preferably, the pressure and time are sufficient to deform the fibers and substantially eliminate the voids, and more preferably the pressure and time are sufficient to form a translucent film.

This process for forming the piece 3 can be described is follows: The fibers, particularly ECPE fibers of the type disclosed in US-A-4 413 110; with a weight average molecular weight of at least 500,000 preferably, a tenacity of at least 6.2 dN/tex (7 g/denier) and a tensile modulus of at least 441.5 dN/tex (500 g/d), are formed into a network such as by weaving or its equivalent including tabby weave, basket weave and satin weave; although other more elaborate weaves such as triaxial weaves, or knits or non-wovens may also be used.

It is contemplated that either the multifilament yarns used in preparing the fabrics or the fabrics themselves may be heat set or heat shrunk (in the presence of applied pressure) prior to the practice of the present invention.

In one present process, pressure is applied to the fibrous network at a temperature between 100°C and 160°C, with the pressure and time being sufficient to achieve a film-like article which is substantially free of voids, and especially has a nigh degree of resistance to puncture.

The temperature of the process may vary from 100°C to 160°C, with 110°C to 155°C being preferred. For some applications, the range of 140°C to 155°C is more preferred. It is considered particularly surprising that those higher temperatures are effective and do not substantially degrade film properties given that the polyethylene used generally has a melting temperature of approximately 138°C (by DSC at 10°/min) and the fiber a main melting temperature of 144-158° C, depending upon the mode of preparation.

In the course of this process, it is important that the melting point be exceeded for a time so that the fibers can be pressed to flow them into a sheet-like substantially impervious material.

When these oriented fibers are shrunk under the specified processing conditions they produce a relatively flexible material which is highly resistant to puncture.

More importantly it is preferred that the pressure and temperature and time of the processing conditions are selected such that at least 55% of the tensile strength of the fiber is retained, preferably 60 through 90 more preferably 60 through 80%.

The protective patch of this invention is made by adhering a previously cut puncture resistant piece 3 to the adhesive side of normal adhesive surgical tape 2 either using the adhesive already on the tape or a special adhesive to provide additional adhering action between the tape and the puncture resistant piece.

Rectangular patches typically can range in length from 4.4 to 7.6 cm (1 3/4 to 3 inches) in length and have a width of 0.6 to 2.5 cm (1/4 inch to 1 inch). The protective piece can range from 0.6 to 2.5 cm) (1/4 to 1 inch) wide 2.5-5.0 cm (1-2 inches) long in the rectangular cenfiguration. When a round patch is used the diameter of the patch can typically range from 2.5 to 10 cm (1 to 4 inches) and the protective piece from 1.9 to 7.6 cm (3/4 to 3 inches). Other shapes and dimensions may also be used.

The puncture resistance of the protective piece 3 should be at least 13.4 kg/cm (75 lbs./in.) and preferably 22.3 to 26.8 kg/cm (125 to 150 lbs./in.) or more when tested as provided herein. It is also preferred that the piece 3 be flexible, and in this respect will generally have a thickness from about 0.005-0.038cm (0.002-0.015 inches).

In practice the patch of any suitable configuration is adhered to vulnerable portions of the body such as fingers or thumbs to provide protection during surgery from puncture due to the use of scalpels, syringes, sutures, etc. Likewise, it could be used by other individuals whose job brings them in to contact with sharp, pointed materials that are likely to puncture or cut the skin.

Referring now to Table 1 below the products that comprise the present invention including PET film and ECPE fiber composite film are compared to other gloves currently on the market. The first column is the thickness of the surface to be penetrated. The second column represents the actual "Puncture Force" in pounds that it takes to pierce the specimen, and the third column is the "Normalized Puncture Force" expressed in pounds of force required to pierce the specimen divided by the thickness thereof. As can be seen, the PET, polycarbonate, and ECPE fiber composite materials were substantially superior in Normalized Puncture Resistance when compared to existing materials.

**TABLE I**

| PUNCTURE RESISTANCE OF VARIOUS PRODUCTS | | | | | | |
|---|---|---|---|---|---|---|
| **MATERIALS** | **THICKNESS** | | **PUNCTURE FORCE** | | **NORMALIZED PUNCTURE** | |
| | **(mil)** | **mm** | **(lbs.)** | **kg** | **(lbs/in)** | **kg/cm** |
| Surgical Latex Glove (Single Layer) | (7) | 1.8 | (0.298) | 0.13 | (42.6) | 7.6 |
| Surgical Latex Glove (double layer) | (14) | 3.6 | (0.587) | 0.26 | (42.0) | 7.5 |
| Playtex House Glove | (26) | 6.6 | (0.26) | 0.18 | (10.0) | 1.8 |
| Leather Golf Glove | (16) | 4.1 | (0.736) | 0.33 | (46.0) | 8.2 |
| Leather Work Glove | (54) | 13.7 | (1.35) | 0.61 | (25.0) | 4.5 |
| Neoprene Coated Fabric | (51) | 13.0 | (3.06) | 1.38 | (60.0) | 10.7 |
| Nylon PET Film Type D | (5) | 1.3 | (0.87) | 0.39 | (174.0) | 31.1 |
| | (10) | 2.5 | (2.33) | 1.05 | (233.0) | 41.6 |
| ICI PET Film | (5) | 1.3 | (0.706) | 0.32 | (141,0) | 25.2 |
| | (10) | 2.5 | (2.31) | 1.04 | (231.0) | 41.3 |
| Poly Carbonate | (5) | 1.3 | (0.7) | 0.31 | (140.0) | 25.0 |
| | (15) | 2.5 | (2.92) | 1.31 | (195.0) | 34.8 |
| UHMWPE | (10) | 1.3 | (0.65) | 0.29 | (65.0) | 11.6 |
| ECPE Fiber Composite | (11) | 2.8 | (2.00) | 0.90 | (192.0) | 34.3 |

To be efficient, it is important that the puncture resistance of the piece be at least 13.4 kg/cm (75 pounds per inch) of thickness of the piece (lbs/in) preferably at least 22.3 kg/cm (125 lbs./in), more preferably at least 26.8 kg/cm (150 lbs/in), when tested by the test method specified below.

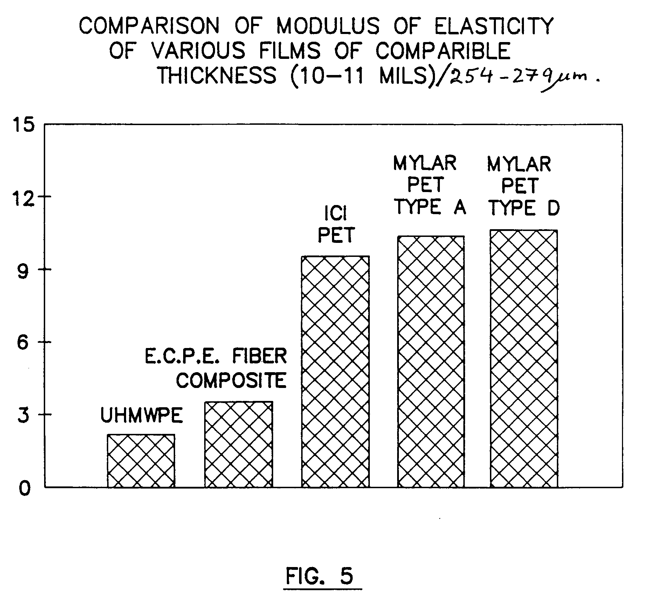

Another important fact is the flexibility of the piece 3. Referencing Fig. 5 it in noted that the ECPE composite film of the invention is substantially superior to PET and polycarbonate.

From the above it can be seen that products of this invention provide superior puncture resistance when compared to other materials; and in the case of ECPE fibers composite film of improved flexibility versus other substantially puncture resistant materials such as polyester and polycarbonate.

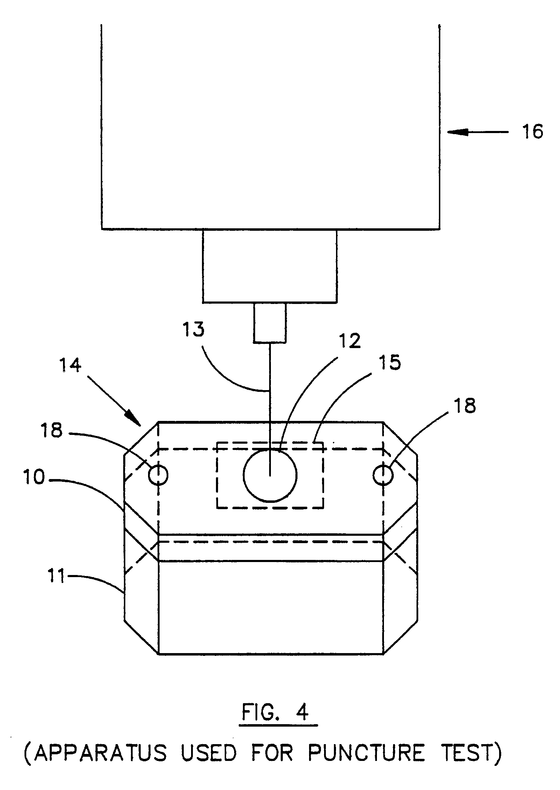

The test method used for Figure 4 was ASTM D-790-86 method 1. The particular property measured was the tangent modulus of elasticity, test method 1 which is a measure of the stiffness of the material, i.e. the greater the number the stiffer the material. The test procedure was modified slightly because of load cell insensitivity. Sample size was 10 x 10 cm (4 x 4 inches) square and the heat speed was 25.4 cm (10 inches) per minute. Also because of the flexibility of the material being tested it was not possible to achieve a rupture or maximum fiber strength of 5% per section 3.2. Accordingly, the specimen was deflected just enough to get a representative slope of the stress/strain curve.

### TESTING PROCEDURE FOR PUNCTURE

Referring to Fig. 4 the apparatus used for the puncture rest essentially comprises an assembly of two flat metal plates 10, 11 between which the material that is to be tested is clamped A circular opening 12 of approximately 7.9 cm (three 1/8") in diameter in the middle of the plates 10,11 serves as the penetration area for the needle 13 such as conventional medical K-wire. The clamp assembly 14 containing the fabric 15 is placed underneath the moving head of an Instron tensile tester 16 to which K-wire 13 is attached through a needle holder and adapter on the head of the Instron 16. The downward movement of the cross hand enables the penetration of the needle into the fabric at a specific speed. The K-wire has a shank diameter of about 0.089 cm (0.035 inches). The test specimen 15 must be large enough so that the clamping bolts 18 securely hold the specimen and restrict any fabric movement during needle penetration It must have an aea in excess of 161.25 cm² of (25 square inches). In practice, the clamp assembly 14 is moved into position to secure the test specimen tightly and the cross-head containing the K-wire moves down at a speed of 5 inches per minute.

### NEEDLE SELECTION

Resistance to puncture is not only a material function out also a function of the needle "sharpness". The sharpness of the needle is influenced by its manufacturing process since very tight tolerances are needed. Because of the variation in "sharpness" of various needles, it is necessary to select those that exhibit the same sharpness against penetration into a given material so that a meaningful comparison of various material is achieved. The needle "sharpness" is determined by measuring the force needed for its penetration into a 2.54 mm (20 mil) thick ultra high molecular weight (UHMW) polyethylene (PE) sheet formed by applying heat and pressure to UHMWPE granules between two endless belts which applying heat and pressure. Similar to the fabric that is to be tested, a UHMW specimen is mounted in the support assembly and each needle is penetrated once into the sheet and its normalized puncture value is determined. From the many different needles tested, we can conclude that those needles exhibiting puncture values between 11.5 and 14.8 kg/cm (65 and 83 lbs/in) have the same "sharpness" (this range is taken as plus or minus one Coefficient of Variation (CV) around the mean).

### PROCEDURE

1. Pre-screened needles which show a puncture value between 11.6 and 1.8 kg/cm (65 and 83 lbs/in) are selected for the test.
2. The test specimen 15 is securely clamped in the support assembly 14 and placed beneath the moving head of the Instron tensile tester 16.
3. The distance from the tip of the needle to the fabric or/film should be maintained at 0.3 to 0.6 cm (1/8 to 1/4 inch).
4. The needle penetrates the fabric at a constant speed of 10 cm (5 inches) per minute.
5. For each specimen, ten penetrations with the same needle are used and averaged cut.

### REPORT:

1. Normalized Maximum Puncture value is reported in lbs/in. of specimen thickness). This value is the maximum force necessary to puncture the material and is normalized by the specimen thickness. It represents the necessary force for complete needle penetration into the fabric.
   a) Measure the fabric thickness by micrometer;
   b) Determine the average puncture force from ten test replicates;
   c) Report the results in lbs/in.
2. The initial slope (force vs needle movement).
3. The Yield point. This is the first puncture point, i.e., the point at which the tip of the needle has just penetrated the specimen. For samples in which do not exhibit a distinguished Yield Point, it is taken as the point in which the initial slope of the curve changes by 70%.
4. Maximum distance of specimen deflection before rupture penetration.

The above test results are important parameters in comparing the behavior of various materials in a puncture test. However, the most important value determining the puncture resistance is the normalized Puncture force reported in lbs/in.

With reference to Table 1 above it is noted that the flexible poly(ethylene terephthalate) film having a thickness of 0.0254 cm (0.010 inches) provides the best puncture resistance of many fabrics expressed as normalized puncture resistance (lbs./in.).

The PET specimen represents biaxially oriented film having a thickness of 2.5 mm. (10 mils) and 1.2 mm (5 mils).

With reference to Table 1 above it is noted that the poly(ethylene terephthalate) film having a thickness of about 0.0254 cm (0.010) inches provides the best puncture resistance of the fabric thickness expressed as normalized puncture (lbs./inch) of protective piece thickness. This film in generally biaxially oriented for best results.

The polycarbonate specimen represents polycarbonate film having a thickness of 0.013 and 0.038 cm (0.005 and .015 inches).

The ECPE composite fiber specimen represents a previously formed network of ECPE fibers as indicated in a preferred embodiment above.

The UHMW specimen represents an ultra high molecular weight PE film material manufactured by applying heat and pressure to conventional ultra high molecular weight polyethylene to form it into a film between two opposed endless belts which apply heat and pressure to the powder to form the film.

From this Table it can also be seen that it is particularly important that the protective piece be formed from a thin, tough puncture resistance flexible material.

An can be seen from Table 1 above, polycarbonate, PET film and ECPE fibers composite specimens ere substantially superior to the plain ultrahigh molecular weight polyethylene specimen. Likewise is noted that they are substantially superior in puncture resistance to conventional gloves.

## Claims

1. A protective patch having an adhesive tape (2) and a needle puncture-resistant piece (3) affixed to the tape whereby the patch may be adhered to a portion of the body vulnerable to cuts and punctures, characterized in that the puncture-resistant piece comprises a network of extended chain polyethylene (ECPE) fibers which have been subjected to heat and pressure for a time sufficient to cause adjacent fibers to melt and adhere to form an article substantially free of voids.

2. The protective patch of claim 1 wherein the puncture resistant piece is made from ECPE fibers having an average molecular weight of at least 500,000, a tenacity of at least 17.7 dN/tex (20 grams per denier), and a tensile modulus of at least 441.5 dN/tex (500 grams per denier).

3. The protective patch of claim 1 wherein the puncture resistant piece has a thickness ranging from 0.05-0.37 mm (0.002-0.015") and is flexible enough to allow conformity of the patch to portions of human body.

4. The protective patch of claim 1 wherein the puncture-resistant piece has a Modulus of Elasticity as measured by ASTM D-790-86 of less than 0.84 x 10⁵ kg/cm² (12 X 10⁵ PSI).

5. The protective patch of claim 1 wherein the puncture-resistant piece has a normalized puncture resistance to a needle traveling at a constant speed of 12.7 cm (5 inches) per minute of at least 13.4 kg/cm (75 lbs./in).

6. The protective patch of claim 4 wherein the modulus of elasticity is less than 0.42 x 10⁵ kg/cm² (6 x 10⁵ PSI).

7. The protective patch of claims 5 wherein the puncture-resistant piece has a normalized puncture resistance of at least 22.3 kg/cm (125 lbs./in).

8. The protective patch of claim 7 wherein the puncture-resistant piece has a normalized puncture resistance of at least 26.8 kg/cm (150 lbs/in).

## Patentansprüche

1. Schutzpflaster mit einem Klebeband (2) und einem nadeldurchstoßfesten Stück (3), das an dem Klebeband befestigt ist, mit dem das Pflaster auf einem durch Schnitte und Stiche gefährdeten Körperteil haftend aufgebracht werden kann, dadurch gekennzeichnet, daß es sich bei dem durchstoßfesten Stück um ein Netz aus Fasern aus kettenverlängertem Polyethylen (ECPE), die so lange mit Hitze und Druck behandelt werden, daß benachbarte Fasern schmelzen und aneinander haften, wobei ein im wesentlichen hohlraumfreier Gegenstand entsteht, handelt.

2. Schutzpflaster nach Anspruch 1, wobei das durchstoßfeste Stück aus ECPE-Fasern mit einem mittleren Molekulargewicht von mindestens 500.000, einer Feinheitsfestigkeit von mindestens 17,7 dN/tex (20 Gramm pro Denier) und einem Zugmodul von mindestens 441,5 dN/tex (500 Gramm pro Denier) besteht.

3. Schutzpflaster nach Anspruch 1, wobei das durchstoßfeste Stück 0,05-0,37 mm (0,002-0,015 Zoll) dick und so flexibel ist, daß sich das Pflaster an Teile des menschlichen Körpers anschmiegen kann.

4. Schutzpflaster nach Anspruch 1, wobei das durchstoßfeste Stück einen Elastizitätsmodul gemäß ASTM D-790-86 unter 0,84 x 10⁵ gk/cm² (12 x 10⁵ psi) aufweist.

5. Schutzpflaster nach Anspruch 1, wobei das durchstoßfeste Stück gegenüber einer sich mit einer konstanten Geschwindigkeit von 12,7 cm (5 Zoll) pro Minute bewegenden Nadel eine normierte Durchstoßfestigkeit von mindestens 13,4 kg/cm (75 lb/Zoll) aufweist.

6. Schutzpflaster nach Anspruch 4, wobei das Elastizitätsmodul unter 0,42 x 10⁵ kg/cm² (6 x 10⁵ psi) liegt.

7. Schutzpflaster nach Anspruch 5, wobei das durchstoßfeste Stück eine normierte Durchstoßfestigkeit von mindestens 22,3 kg/cm (125 lb/Zoll) aufweist.

8. Schutzpflaster nach Anspruch 7, wobei das durchstoßfeste Stück eine normierte Durchstoßfestigkeit von mindestens 26,8 kg/cm (150 lb/Zoll) aufweist.

## Revendications

1. Pansement protecteur possédant un ruban adhésif (2) et un morceau résistant aux piqûres d'aiguilles (3) fixé sur le ruban, permettant de coller le pansement sur une partie du corps vulnérable aux coupures et aux piqûres, caractérisé en ce que le morceau résistant aux piqûres comprend un réseau de fibres de polyéthylène à chaîne allongée (ECPE) ayant été soumises à de la chaleur et une pression pendant suffisamment longtemps pour que les fibres adjacentes fondent et se collent pour former un article essentiellement exempt de vides.

2. Pansement protecteur selon la revendication 1, dans lequel le morceau résistant aux piqûres est fait de fibres de ECPE ayant une masse moléculaire moyenne d'au moins 500 000, une ténacité d'au moins 17,7 dN/tex (20 grammes par denier) et un module de traction d'au moins 441,5 dN/tex (500 grammes par denier).

3. Pansement protecteur selon la revendication 1, dans lequel le morceau résistant aux piqûres a une épaisseur s'échelonnant de 0,05 à 0,37 mm (0,002-0,015") et est suffisamment souple pour que le pansement épouse la forme de parties du corps humain.

4. Pansement protecteur selon la revendication 1, dans lequel le morceau résistant aux piqûres a un module d'élasticité, mesuré par ASTM D-790-86, inférieur à 0,84 x 10⁵ kg/cm² (12 x 10⁵ psi).

5. Pansement protecteur selon la revendication 1, dans lequel le morceau résistant aux piqûres a une résistance aux piqûres normalisée, à une aiguille se déplaçant à une vitesse constante de 12,7 cm (5 pouces) par minute, d'au moins 13,4 kg/cm (75 lbs/in).

6. Pansement protecteur selon la revendication 4, dans lequel le module d'élasticité est inférieur à 0,42 x 10⁵ kg/cm² (6 x 10⁵ psi).

7. Pansement protecteur selon la revendication 5, dans lequel le morceau résistant aux piqûres a une résistance aux piqûres normalisée d'au moins 22,3 kg/cm (125 lbs/in).

8. Pansement protecteur selon la revendication 7, dans lequel le morceau résistant aux piqûres a une résistance aux piqûres normalisée d'au moins 26,8 kg/cm (150 lbs/in).
